(19) Europäisches Patentamt
European Patent Office
Office européen des brevets

(11) Numéro de publication: **0 006 772**
**B1**

(12) # FASCICULE DE BREVET EUROPEEN

(45) Date de publication du fascicule de brevet: **04.11.81**

(21) Numéro de dépôt: **79400282.4**

(22) Date de dépôt: **02.05.79**

(51) Int. Cl.³: **C 07 D 491/048,**
C 07 D 495/04,
A 61 K 31/44
//(C07D491/048, 307/00,
221/00), (C07D495/04,
307/00, 221/00)

(54) **Dérivés de la thiéno et furopyridone, procédé pour leur préparation et médicaments les contenant.**

(30) Priorité: **22.06.78 FR 7818684**

(43) Date de publication de la demande:
**09.01.80 Bulletin 80/1**

(45) Mention de la délivrance du brevet:
**04.11.81 Bulletin 81/44**

(84) Etats Contractants Désignés:
**DE NL**

(56) Documents cités:
**FR - A - 2 115 006**
**FR - A - 2 385 720**

(73) Titulaire: **OMNIUM FINANCIER AQUITAINE POUR L'HYGIENE ET LA SANTE - SANOFI**
**Tour Aquitaine Cédèx No 4**
**F-92080 Paris La Defense (FR)**

(72) Inventeur: **Maffrand, Jean-Pierre**
**5, rue du Corps Franc Pommiès**
**F-31120 Portet sur Garonne (FR)**

(74) Mandataire: **Moncheny, Michel et al,**
**c/o Cabinet Lavoix 2 Place d'Estienne d'Orves**
**F-75441 Paris Cedex 09 (FR)**

**EP 0 006 772 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

Dérivés de la thiéno et furopyridone, procédé pour leur préparation et médicaments les contenant

La présente invention est relative à un médicament contenant à titre de principe actif des dérivés de la thiéno et de la furopyridone.

Elle ainsi pour objet un médicament ayant notamment des activités inhibitrices de l'agrégation plaquettaire et anti-inflammatoire, caractérisé en ce qu'il contient, à titre de principe actif, une quantité efficace d'un dérivé de thiénopyridone ou de furopyridone répondant aux formules générales suivantes:

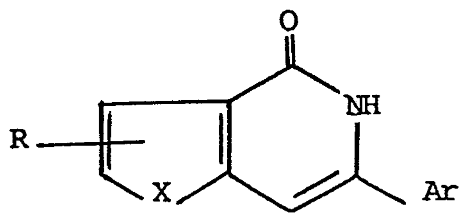

(I)

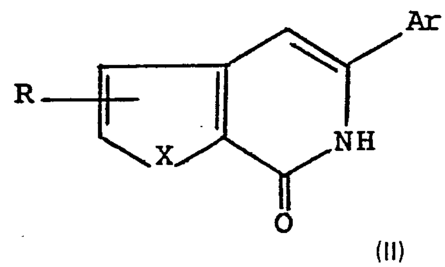

(II)

dans lesquelles X est un atome d'oxygène ou de soufre; R représente un atome d'hydrogène ou d'halogène ou un groupe alcoyle en $C_1$ à $C_4$ et Ar représente un groupe phényle, naphtyle ou pyridyle, éventuellement substitué par au moins un atome d'halogène ou un groupe alcoyle inférieur, alcoxy inférieur, hydroxy, trifluorométhyle ou nitro.

De tous les composés de formule générale I ou II seul le composé de formule II dans laquelle R = H, X = S et Ar = $C_6H_5$, n'est pas nouveau. Il a déjà été signalé dans la littérature (D.E. AMES et O. RIBEIRO, J.C.S. Perkin I, 1975, 1390), mais il n'a fait l'objet d'aucune étude thérapeutique et son procédé d'obtention est relativement complexe.

L'invention a donc encore pour objet des dérivés de la thiéno ou de la furopyridone répondant aux formules générales suivantes:

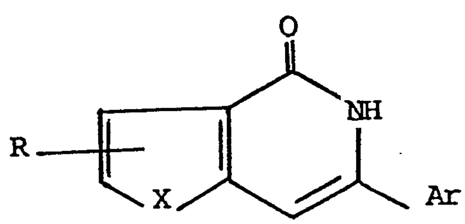

(I)

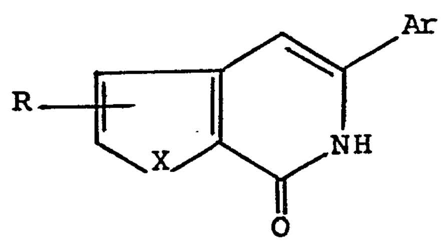

(II)

dans lesquelles X est un atome d'oxygène ou de soufre, R représente un atome d'hydrogène ou d'halogène ou un groupe alcoyle en $C_1$ à $C_4$ et Ar représente un groupe phényle, naphtyle ou pyridile, éventuellement substitué par au moins un atome d'halogène ou un groupe alcoyle inférieur, alcoxy inférieur, hydroxy, trifluorométhyle ou nitro, sous réserve que l'on n'ait pas simultanément R = H, X = S et Ar= phényle.

L'invention a également pour objet un procédé de préparation des dérivés de formule I ou II, caractérisé en ce qu'on traite par chauffage dans un solvant inerte et à une température comprise entre 150 °C et le point d'ébullition du solvant choisi, un composé de formule III ou IV suivante:

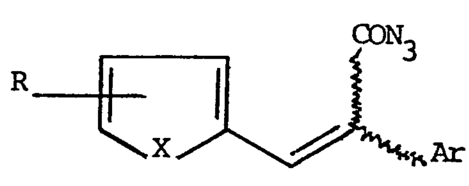

ou

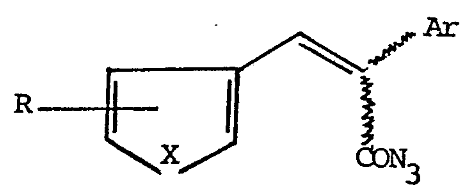

(III)

(IV)

dans lesquelles X, R et Ar sont tels que définis ci-dessus.

Le solvant inerte vis-à-vis des réactifs et des produits réactionnels est par exemple le diphénylméthane ou l'éther diphénylique. Cette réaction par traitement thermique fait intervenir la transposition des azides de formule III ou IV en isocyanates de formule V ou VI qui sont cyclisés thermiquement en dérives I et II, suivant le schéma réactionnel ci-dessous:

(III)

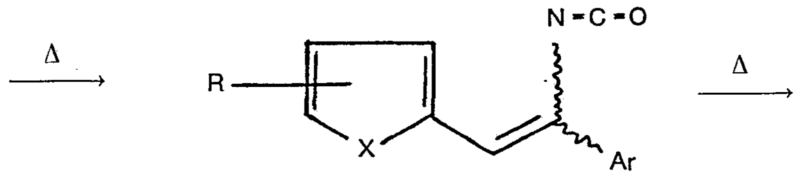


(I)

(V)

(IV)

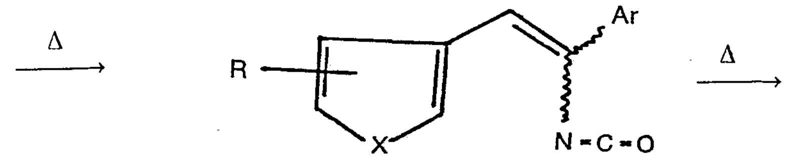


(II)

(VI)

Les composés de départ de formules III et IV sont préparés à partir des acides de formules VII et VIII correspondants qui sont tout d'abord transformés en chlorures d'acides de formules IX et X ou anhydrides mixtes de formules XI et XII, ces derniers étant à leur tour transformés de façon classique en azides, par exemple par réaction avec l'azide de sodium en solution aqueuse, suivant le schéma réactionnel ci-après:

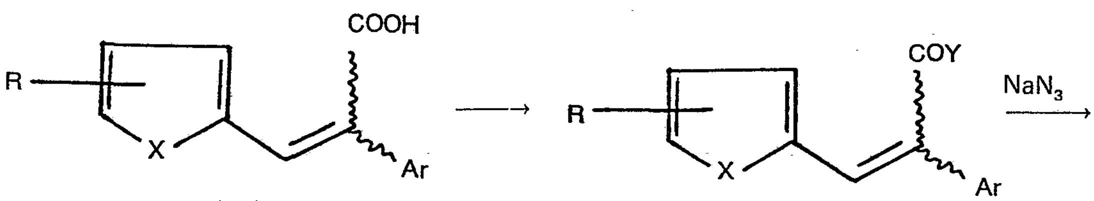


(III)

(VII)

Y = CL: (IX)
Y = $OCOOC_2H_5$: (XI)

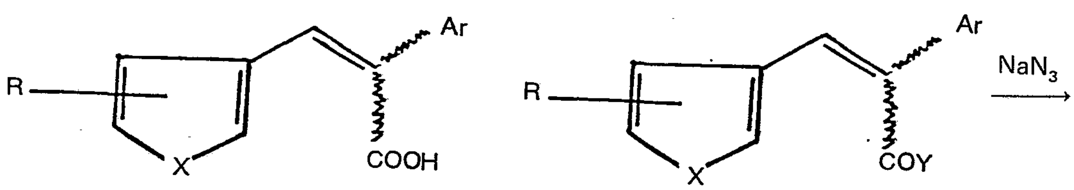


(IV)

(VIII)

Y = Cl: (X)
Y = $OCOOC_2H_5$: (XII)

Les acides de formule VII suivants, dans lesquels: X = S, R = H, Ar = $C_6H_5$; X = S, R = H, Ar = 4-Cl-$C_6H_4$; X = S, R = H, Ar = 4-$CH_3$-$C_6H_4$; X = S, R = H, Ar = 4-$NO_2$-$C_6H_4$ et X = O, R = H, Ar = $C_6H_5$, sont décrits dans la littérature: S. FISICHELLA, G. SCARLATA et D. SCIOTTO, Ann.Chim. (Italia), 1973, *63*, 55; S. FISICHELLA, G. MINERI, G. SCARLATA et D. SCIOTTO, ibid. 1973, *63*, 779.

Les autres acides de formules (VII) et (VIII) qui sont utilisés dans le procédé de l'invention sont nouveaux et ils ont été préparés par le procédé décrit dans les articles mentionnés ci-dessus, selonle schéma réactionnel suivant:

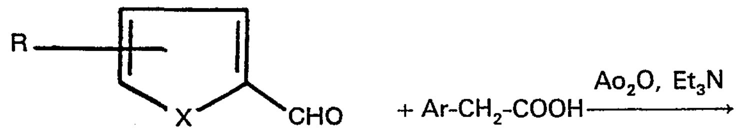


(VII)

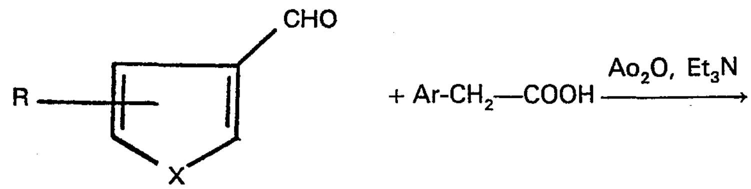


(VIII)

Les exemples non limitatifs suivants sont donnés à titre d'illustration du procédé de préparation des dérivés de l'invention:

Exemple 1

Phényl-6-thiéno(3,2-c)pyridone-4 (dérivé 1)

*a)* Préparation de l'azide de formule III

Une solution refroidie à —5 °C de 50 g (0,217 mole) d'acide $\alpha$-phényl $\beta$-(thiényl-2)acrylique dans 31 cm$^3$ (0,22 mole) de triéthylamine et 350 cm$^3$ de chloroforme, on ajoute, goutte à goutte 21 cm$^3$ ($\sim$ 0,22 mole) de chloroformiate d'éthyle. Après agitation pendant une demi-heure à —5 °C on introduit, goutte à goutte, une solution de 17 g (0,26 mole) d'azide de sodium dans 60 cm$^3$ d'eau. L'introduction terminée, on agite à 0 °C pendant une heure, on ajoute de l'eau et on décante. La phase organique est lavée à l'eau puis séchée sur sulfate de sodium et filtrée.

*b)* Préparation du dérivé de formule I

Le filtrat obtenu à l'etape a) est ajouté, goutte à goutte, à 200 cm$^3$ de diphénylméthane préalablement chauffé à 160 °C, le chloroforme étant distillé au fur et à mesure. L'introduction terminée, on agite encore 15 minutes à la même température, on refroidit, on ajoute de l'éther diisopropylique, on filtre, on lave avec le même éther et on sèche sous vide.

On obtient 31.55 g (Rdt: 63,5%) de cristaux beiges: F = 230 °C que l'on peut recristalliser dans un mélange méthanol-diméthylformamide.

Exemple 2

p-fluorophényl-6 thiéno(3,2-c)pyridone-4, (dérivé 2)

Préparée selon le mode opératoire de l'exemple 1 à partir de l'acide $\alpha$-(fluoro-4 phényl $\beta$-(thiényl-2)acrylique (F = 191 °C): Cristaux beiges, F = 262 °C (méthanol-diméthylformamide) rendement: 44,5%.

Exemple 3

o-chlorophényl-6 thiéno(3,2-c)pyridone-4, (dérivé 3)

Préparée selon le mode opératoire de l'exemple 1 à partir de l'acide $\alpha$-(chloro-2 phényl) $\beta$-(thiényl-2)acrylique (F = 197 °C): Cristaux beiges, F = 232 °C, rendement: 27%.

Exemple 4

p-chlorophényl-6 thiéno(3,2,-c)pyridone-4, (dérivé 4)

Préparée selon le mode opératoire de l'exemple 1 à partir de l'acide $\alpha$ (chloro-4 phényl) $\beta$-(thiényl-2)acrylique (F = 224 °C): Cristaux vert pâle, F > 260 °C, rendement: 60%.

Exemple 5

p-tolyl-6 thiéno(3,2-c)pyridone-4, (dérivé 5)

Préparée selon le mode opératoire de l'exemple 1 à partir de l'acide $\alpha$-(p-tolyl) $\beta$-(thiényl-2) acrylique (F = 209 °C): Cristaux jaunes, F > 260 °C (méthanol-diméthylformamide), Rdt: 51%.

Exemple 6

p-methoxyphényl-6 thiéno(3,2-c)pyridone-4, (dérive 6)

Préparée selon le mode opératoire de l'exemple 1 à partir de l'acide $\alpha$-(méthoxy-4 phényl) $\beta$-(thiényl-2)acrylique (F = 203 °C): Cristaux verdâtres, F = 251 °C (diméthylformamide), rendement: 64%.

Exemple 7

(diméthoxy-3,4 phényl)-6 thiéno (3,2-c)pyridone-4, (dérivé 7)

Préparée selon le mode opératoire de l'exemple 1 à partir de l'acide α-(diméthoxy-3,4 phényl) β-(thiényl-2)acrylique (F = 220 °C): Cristaux jaunes, F = 220 °C (méthanol-diméthylformamide, Rdt: 60,5 %)

Exemple 8

(triméthoxy,3,4,5 phényl)-6 thiéno(3,2-c)pyridone-4, (dérivé 8)

Préparée selon le mode opératoire de l'exemple 1 à partir de l'acide α-(triméthoxy-3,4,5 phényl) β-(thiényl-2)acrylique (F = 217 °C): Cristaux jaunes, F = 240 °C (méthanol-diméthylformamide), Rdt: 62 %.

Exemple 9

o-nitrophényl-6 thiéno(3,2-c)pyridone-4, (dérivé 9)

Préparée selon le mode opératoire de l'exemple 1 à partir de l'acide α-(nitro-2 phényl) β-(thiényl-2)acrylique (F = 211 °C): Cristaux jaunes, F = 236 °C (méthanol-diméthylformamide), Rdt: 20 %.

Exemple 10

p-nitrophényl-6 thiéno(3,2-c)pyridone-4, (dérivé 10)

Préparée selon le mode opératoire de l'exemple 1 à partir de l'acide α-(nitro-4 phényl) β-(thiényl-2)acrylique (F = 236 °C): Cristaux jaunes, F > 260 °C, rendement: 51 %.

Exemple 11

m-trifluorométhylphényl-6 thiéno(3,2-c)pyridone-4 (dérivé 11)

Préparée selon le mode opératoire de l'exemple 1 à partir de l'acide α-(trifluorométhyl-3 phényl) β-(thiényl-2) acrylique (F = 232 °C) Cristaux blancs, F = 245 °C (méthanol-diméthylformamide), Rdt: 58 %.

Exemple 12

Chloro-2 phényl-6 thiéno(3,2-c)pyridone-4, (dérivé 12)

Préparée selon le mode opératoire de l'exemple 1 à partir de l'acide α-phényl β-(Chloro-5 thiényl-2)acrylique (F = 215 °C) Cristaux orangés, F > 260 °C (diméthylformamide), Rdt: 64 %.

Exemple 13

Méthyl-2 phényl-6 thiéno(3,2-c)pyridone-4, (dérivé 13)

Préparée selon le mode opératoire de l'exemple 1 à partir de l'acide α-phényl β-(méthyl-5 thiényl-2)acrylique (F = 207 °C) Cristaux beiges, F > 260 °C (diméthylformamide), Rdt: 54 %.

Exemple 14

(Pyridyl-3)-6 thiéno(3,2-c)pyridone-4, (dérivé 14)

Préparée selon le mode opératoire de l'exemple 1 à partir de l'acide α-(pyridyl-3) β-(thiényl-2)acrylique (F = 210 °C) Cristaux jaunes, F > 260 °C (diméthylformamide), rendement: 41 %.

Exemple 15

Phényl-5 thiéno(2,3-c)pyridone-7, (dérivé 15)

Préparée selon le mode opératoire de l'exemple 1 à partir de l'acide α-phényl β-(thiényl-3)acrylique (F = 175 °C) Cristaux rosés, F = 203°C méthanol-diméthylformamide), Rdt: 50 %.

Exemple 16

o-chlorophényl-5 thiéno(2,3-c)pyridone-7, (dérivé 16)

Préparée selon le mode opératoire de l'exemple 1 à partir de l'acide α-(chloro-2-phényl) β-(thiényl-3)acrylique (F = 196 °C) Cristaux beiges, F = 229 °C, rendement: 39 %.

Exemple 17

Phényl-6 furo(3,2-c)pyridone-4, (dérivé 17)

Préparée selon le mode opératoire de l'exemple 1 à partir de l'acide α-phényl β-(furyl-2)acrylique (F = 146 °C): Cristaux beiges, F = 210 °C, rendement: 65 %.

Exemple 18

α-naphtyl-6 furo(3,2-c)pyridone-4

Préparée selon le mode opératoire de l'exemple 1 à partir de l'acide α-(naphtyl-1) β-(furyl-2)acrylique (F = 219 °C). Cristaux beiges, F = 230 °C, rendement: 39,5 %.

Les résultats des essais toxicologiques et pharmacologiques qui sont rapportés ci-après, mettent

en évidence les intéressantes activités des dérivés de l'invention, notamment inhibitrice de l'agrégation plaquettaire et anti-inflammatoire.

I — Etude Toxicologique

Les composés de l'invention bénéficient d'une excellente tolérance et d'une toxicité. Ainsi, la $DL_{50}$/24 h/kg de poids corporel d'animal, déterminée chez la souris selon la méthode de Miller et Tainter pour la voie orale, est supérieure à 400 mg pour tous les dérivés.

En outre, les essais effectués sur la toxicité aigüe, chronique sub-chronique et retardée, chez diverses espèces animales, n'ont mis en évidence aucune réaction locale ou générale, aucune perturbation dans les contrôles biologiques régulièrement effectués, aucune anomalie dans les examens microscopiques et macroscopiques chez les animaux sacrifiés et autopsiés en fin d'expérimentation.

II — Etude Pharmacologique

1°— *Action inhibitrice de l'agrégation plaquettaire*

Chez des rats de souche Wistar, on effectue un prélèvement sanguin dans la veine jugulaire. A partir de ce sang citraté et après centrifugation, on reconstitue un plasma contenant 600 000 ± 20 000 plaquettes par $mm^3$, qui servira dans toutes les mesures d'agrégation.

a) *mesure de l'agrégation plaquettaire à l'A.D.P.*

On place 0,4 ml de plasma dans un tube siliconé pourvu d'une barre aimantée ellemême siliconée. Le tube est introduit dans un agrégomètre couplé à un appareil permettant d'enregistrer les variations de densité optique. Lorsque la transmission de la lumière a atteint une valeur stable, on introduit dans le tube 0,5 ml d'une solution contenant 10 $\mu$M d'A.D.P. (Adénosine-Di Phosphate).

L'agrégation des plaquettes provoque alors une augmentation de la transmission lumineuse suivie d'une diminution consécutive à la phase de désagrégation.

La variation maximale de densité optique ainsi déterminée caractérisé l'intensité de l'agrégation.

b) *mesure de l'agrégation plaquettaire au collagène.*

La solution d'A.D.P. est remplacée par une solution de collagène (extrait de tendons bovins).

c) *résultats.*

Différents lots de 20 rats sont utilisés, chaque lot recevant un dérivé à tester par la voie orale, à la dose de 100 mg/kg de poids corporel. Les résultats obtenus au cours de ces 2 essais sont rapportés dans le Tableau I suivant qui indique le pourcentage d'inhibition de l'agrégation plaquettaire obtenu, par rapport au témoin, 3 heures après le traitement par le médicament de l'invention, dans le test de l'A.D.P. et au collagène.

TABLEAU I

| Traitement | Pourcentage d'inhibition | |
|---|---|---|
| | A.D.P. | Collagène |
| dérivé 1 | 62,0 | 91,4 |
| dérivé 2 | 60,5 | 91,7 |
| dérivé 3 | 58,8 | 92,0 |
| dérivé 4 | 61,2 | 90,5 |
| dérivé 5 | 62,6 | 91,4 |
| dérivé 6 | 62,2 | 91,7 |
| dérivé 7 | 61,7 | 90,6 |
| dérivé 8 | 60,4 | 92,2 |
| dérivé 9 | 59,1 | 90,8 |
| dérivé 10 | 60,4 | 90,2 |
| dérivé 11 | 60,1 | 90,7 |
| dérivé 12 | 60,6 | 91,4 |
| dérivé 13 | 61,5 | 90,5 |
| dérivé 14 | 60,8 | 91,2 |
| dérivé 15 | 62,5 | 90,8 |
| dérivé 16 | 60,1 | 90,2 |
| dérivé 17 | 59,8 | 90,4 |

2° — *Action anti-inflammatoire*

a) *méthode de l'oedème localisé provoqué par la carragénine.*

Une solution de carragénine (0,1 ml) à 1% est injectée dans les fléchisseurs métatarsiens de la patte postérieure droite du rat au temps 0. Les animaux du lot traité reçoivent en outre, par la voie orale, 100

mg par kg du dérivé à tester respectivement 1 heure avant, en même temps que l'injection de l'agent phlogogène, puis 1 heure et 2 heures et demi après. Les mesures qui sont effectuées à l'aide du micromètre de ROCH aux temps 0, 1 heure, 2 heures, 3 heures et 5 heures après l'administration de la carragénine permettent de déterminer, en fonction du temps, le pourcentage d'activité anti-inflammatoire par comparaison avec le lot témoin.

Les résultats sont consignés au tableau II suivant:

TABLEAU II

| Dérivés N° | 1ère heure | 2ème heure | 3ème heure | 5ème heure |
|---|---|---|---|---|
| 1 | 35 | 39 | 45 | 49 |
| 2 | 37 | 41 | 46 | 50 |
| 3 | 37 | 40 | 45 | 48 |
| 4 | 41 | 44 | 48 | 52 |
| 5 | 38 | 41 | 46 | 51 |
| 6 | 40 | 43 | 49 | 52 |
| 7 | 40 | 44 | 48 | 51 |
| 8 | 36 | 39 | 45 | 59 |
| 9 | 38 | 41 | 46 | 50 |
| 10 | 41 | 45 | 51 | 53 |
| 11 | 40 | 44 | 49 | 52 |
| 12 | 36 | 40 | 46 | 50 |
| 13 | 38 | 42 | 47 | 50 |
| 14 | 38 | 41 | 47 | 50 |
| 15 | 39 | 43 | 48 | 51 |
| 16 | 37 | 41 | 47 | 50 |
| 17 | 41 | 44 | 49 | 51 |

b) *méthode de l'oedème généralisé à l'ovalbumine.*

Une injection intrapéritonéale simultanée de 1 ml d'ovalbumine et de 0,5 ml d'une solution aqueuse de bleu Evans à 1 % est effectuée sur le rat. D'autre part, on administre per os aux animaux du lot traité 100 mg/kg du dérivé à tester 1 heure avant et en même temps que l'ovalbumine. L'intensité du phénomène ainsi provoqué est notée par un chiffre allant de 1 à 5 suivant la progression du syndrome inflammatoire. On détermine ainsi la moyenne de l'intensité oedémateuse et le pourcentage de diminution de la réaction oedémateuse par rapport au témoin, en fonction du temps.

Les pourcentages d'activité anti-inflammatoire obtenus à la 2ème heure et 3eme heure après l'injection d'ovalbumine sont consignés dans le Tableau III suivant.

TABLEAU III

| Dérivés N° | Pourcentage d'activité anti-inflammatoire | |
|---|---|---|
| | 2ème heure | 3ème heure |
| 1 | 45 | 52 |
| 2 | 48 | 54 |
| 3 | 48 | 55 |
| 4 | 47 | 53 |
| 5 | 50 | 55 |
| 6 | 52 | 58 |
| 7 | 48 | 55 |
| 8 | 46 | 53 |
| 9 | 46 | 52 |
| 10 | 49 | 55 |
| 11 | 51 | 57 |
| 12 | 45 | 52 |
| 13 | 46 | 53 |
| 14 | 48 | 54 |
| 15 | 47 | 53 |
| 16 | 46 | 54 |
| 17 | 45 | 51 |

Les résultats de ces études mettent en évidence la bonne tolérance et les intéressantes propriétés inhibitrice de l'agrégation plaquettaire et anti-inflammatoire des dérivés de formules I et II, qui les rendent très utiles en médecine humaine et vétérinaire.

Le médicament de l'invention peut être présenté pour l'administration orale, sous forme de comprimés, comprimés dragéifiés, capsules, gouttes et sirop. Il peut aussi être présenté, pour l'administration rectale, sous forme de suppositoires et pour l'administration parentérale, sous forme de soluté injectable.

Chaque dose unitaire contient avantageusement de 0,040 g à 0,750 g de principe actif, les doses administrables journellement pouvant varier de 0,040 g à 1,50 g de principe actif selon l'âge du patient et l'affection traitée.

On donnera ci-après, à titre d'exemples non limitatifs quelques formulations pharmaceutiques du médicament de l'invention.

1°) Comprimes
dérivé n° 1 0,125 g
excipient: lactose, polyvinylpyrrolidone, acide alginique, fécule de pomme de terre, stéarate de magnésium.

2°) Comprimes Drageifies
dérivé n° 4 0,100 g
excipient: eudragit S, talc, stéarate de magnésium, gomme arabique, sucre blanc officinal, cire blanche, cire de carnauba.

3°) Capsules
dérivé n°6 0,150 g
excipient: stéarate de magnésium, talc, lactose

4°) Ampoules Injectables
dérivé n° 12 0,100 g
excipient: solvant isotonique q.s.p. 3 ml

5°) Suppositoires
dérivé n° 15 0,150 g
excipient: triglycérides semi synthétiques

Les études toxicologiques et pharmacologiques qui viennent d'être rapportées ont mis en évidence la faible toxicité des dérivés de l'invention ainsi que leurs remarquables actions inhibitrices de l'agrégation plaquettaire et anti-inflammatoire.

Le médicament de l'invention peut ainsi être administré à l'homme avec profit, à titre préventif ou curatif, dans le traitement des maladies provoquant une modification pathologique de l'agrégation plaquettaire, telle que les maladies thrombo-emboliques.

Il peut aussi être administré pour ses propriétés anti-inflammatoires et anti-oedémateuses dans le traitement de toutes les réactions inflammatoires sans pour cela interférer sur l'évolution du processus pathologique sous-jacent. Il est indiqué dans la poly-arthrite rhumatoïde, l'arthrose, la coxarthrose, la spondylarthrite ankylosante, la goutte, les affections aiguës de l'appareil locomoteur, en chirurgie post-opératoire et en odonto-stomatologie.

## Revendications

1. Médicament ayant notamment des activités inhibitrices de l'agrégation plaquettaire et anti-inflammatoire, caractérisé en ce qu'il contient, à titre de principe actif, une quantité efficace d'un dérivé de thiénopyridone ou de furopyridone répondant aux formules générales suivantes:

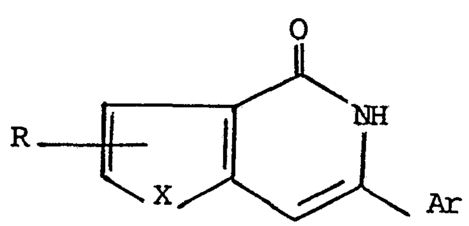

(I)

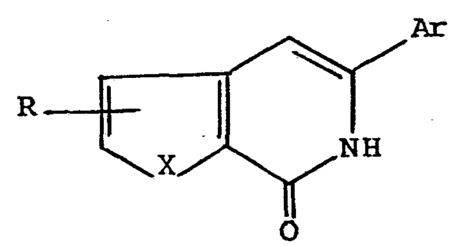

(II)

dans lesquelles X est un atome d'oxygène ou de soufre, R représente un atome d'hydrogène ou d'halogène ou un groupe alcoyle en $C_1$ à $C_4$ et Ar représente un groupe phényle, naphtyle ou pyridile, éventuellement substitué par au moins un atome d'halogène ou un groupe alcoyle inférieur, alcoxy inférieur, hydroxy, trifluorométhyle ou nitro.

2. Médicament selon la revendication 1, caractérisé en ce qu'il est présenté sous une forme

appropriée pour une administration orale, parentérale ou rectale, le principe actif étant associé à un véhicule thérapeutiquement acceptable.

3. Médicament selon l'une quelconque des revendications 1 et 2, caractérisé en ce qu'il est présenté sous forme de doses unitaires contenant chacune de 0,040 à 0,750 g de principe actif.

4. Dérivés de thiéno ou de la furopyridone répondant aux formules générales suivantes:

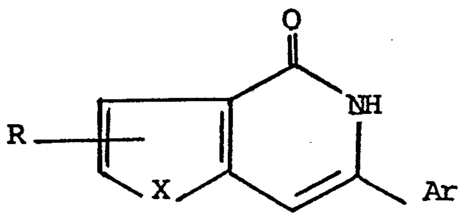

(I)

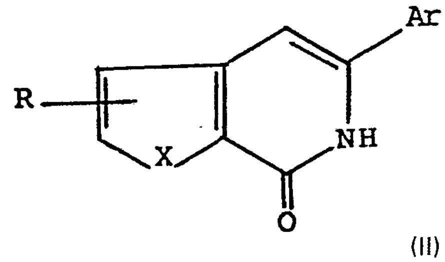

(II)

dans lesquelles X est un atome d'oxygène ou de soufre, R représente un atome d'hydrogène ou d'halogène ou un groupe alcoyle en $C_1$ à $C_4$ et Ar représente un groupe phényle, naphtyle ou pyridyle, éventuellement substitué par au moins un atome d'halogène ou un groupe alcoyle inférieur, alcoxy inférieur, hydroxy, trifluorométhyle ou nitro, sous réserve que l'on n'ait pas simultanément R=H, X=S et Ar=phényle.

5. Procédé de préparation des dérivés de thiénopyridone ou de furopyridone répondant aux formules générales suivantes:

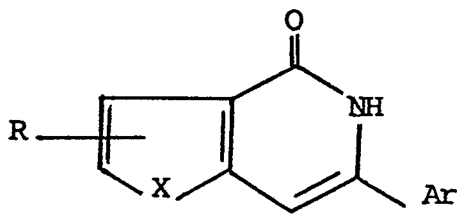

(I)

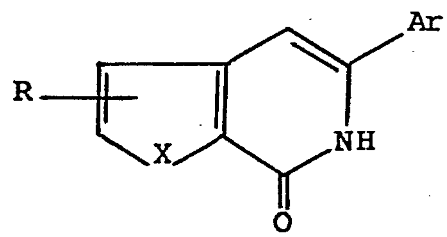

(II)

dans lesquelles X est un atome d'oxygène ou de soufre, R représente un atome d'hydrogène ou d'halogène ou un groupe alcoyle en $C_1$ à $C_4$ et Ar représente un groupe phényle, naphtyle ou pyridyle, éventuellement substitué par au moins un atome d'halogène ou un groupe alcoyle inférieur, alcoxy inférieur, hydroxy, trifluorométhyle ou nitro, caractérisé en ce qu'on traite par chauffage, dans un solvant inerte et à une température comprise entre 150°C et le point d'ébullition du solvant choisi, un composé de formule III et IV suivantes:

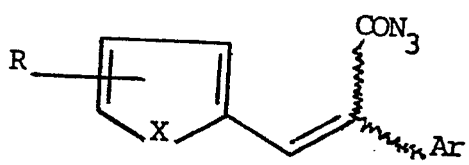

(III)

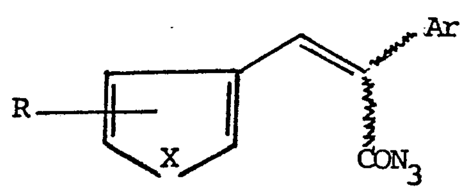

(IV)

dans lesquelles X, R et Ar sont tels que définis ci-dessus.

6. Procédé selon la revendication 5, caractérisé en ce que le solvant inerte est le diphénylméthane ou l'éther diphénylique.

**Patentansprüche**

1. Arzneimittel mit Plättchenaggregation inhibierender und entzündungshemmender Wirkung, dadurch gekennzeichnet, daß es als Wirkstoffkomponente eine wirksame Menge eines Derivates von Thienopyridon oder Furopyridon gemäß der folgenden allgemeinen Formeln:

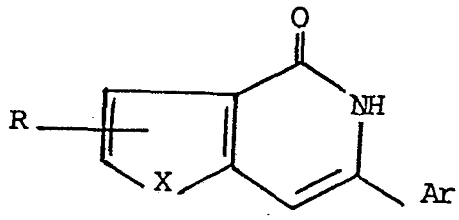

(I)

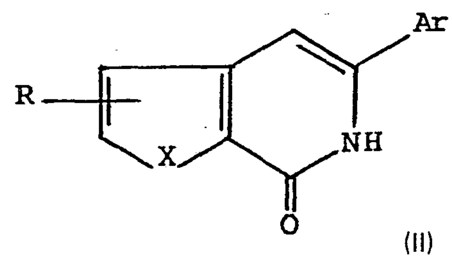

(II)

enthält, in denen X ein Sauerstoff- oder Schwefelatom, R Wasserstoff oder Halogen oder eine Alkylgruppe mit 1 bis 4 Kohlenstoffatomen und Ar eine Phenyl-, Naphthyl- oder Pyridylgruppe, gegebenenfalls substituiert mit mindestens einem Halogenatom oder einer niedrigeren Alkyl- oder Alkoxygruppe, Hydroxy-, Trifluormethyl- oder Nitrogruppe, bedeuten.

2. Arzneimittel gemäß Anspruch 1, dadurch gekennzeichnet, daß es in geeigneter Form zu oraler, parenteraler oder rectaler Verabreichung vorliegt, wobei die aktive Wirkstoffkomponente mit einem therapeutisch verträglichen Trägermaterial vereinigt ist.

3. Arzneimittel nach mindestens einem der Ansprüche 1 und 2, dadurch gekennzeichnet, daß es in einer Einheitsdosis mit Gehalt an 0,040 bis 0,750 g aktiver Wirkstoffkomponente vorliegt.

4. Derivate von Thieno- oder Furopyridon gemäß den folgenden algemeinen Formeln:

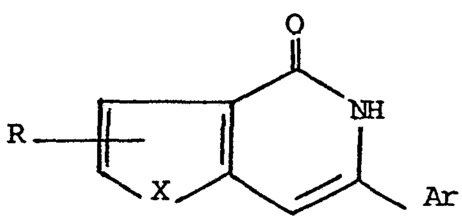

(I)

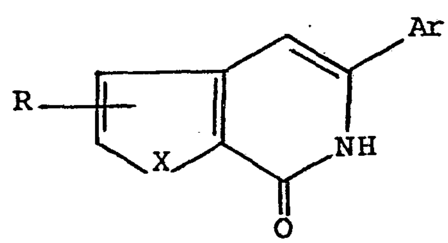

(II)

in denen X Sauerstoff oder Schwefel, R Wasserstoff oder Halogen oder eine Alkylgruppe mit 1 bis 4 Kohlenstoffatomen und Ar eine Phenyl-, Naphtyl- oder Pyridylgruppe, gegebenenfalls substituiert mit mindestens einem Halogenatom oder einer niedrigeren Alkyl, Alkoxygruppe, Hydroxy-, Trifluormethyl- oder Nitrogruppe, bedeuten, mit der Einschränkung, daß nicht gleichzeitig R = H, X = S und AR = Phenyl sind.

5. Verfahren zur Herstellung von Thienopyridon- oder Furopyridonderivaten gemäß den folgenden allgemeinen Formeln:

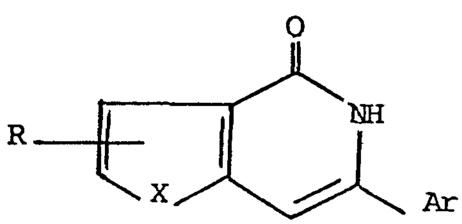

(I)

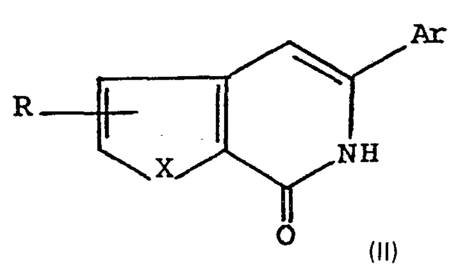

(II)

in denen X Sauerstoff oder Schwefel, R Wasserstoff oder Halogen oder eine Alkylgruppe mit 1 bis 4 Kohlenstoffen und Ar eine Phenyl-, Naphtyl- oder Pyridylgruppe, gegebenenfalls substituiert durch

mindestens ein Halogen oder eine niedrigere Alkyl-, Alkoxygruppe, Hydroxy-, Trifluormethyl- oder Nitrogruppe, bedeuten, dadurch gekennzeichnet, daß man eine Verbindung der folgenden Formeln III oder IV in einem inerten Lösungsmittel in der Hitze bei einer Temperatur zwischen 150°C und dem Siedepunkt des gewählten Lösungsmittels behandelt,

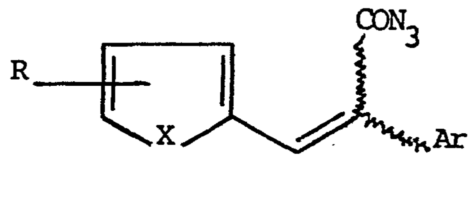

(III)

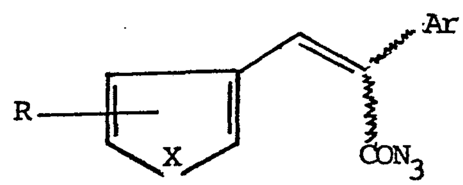

(IV)

wobei, X, R und Ar die zuvor gegebene Bedeutung besitzen.

6. Verfahren nach Anspruch 5, dadurch gekennzeichnet, daß man als inertes Lösungsmittel Diphenylmethan oder Diphenyläther verwendet.

**Claims**

1. Therapeutic composition having, in particular blood-platelet aggregation inhibiting and antiinflammatory activities comprising, as active ingredient, an efficient amount of a thienopyridone or furopyridone derivative having the following general formulae:

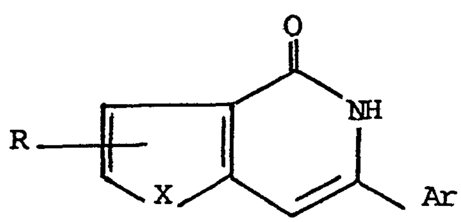

(I)

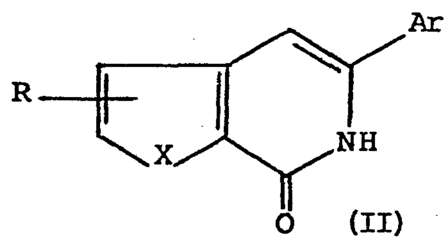

(II)

in which X is an oxygen or sulfur atom, R represents a hydrogen or halogen atom or a $C_{14}$ alkyl group, and Ar represents a phenyl, naphthyl or pyridyl group optionally substituted with at least a halogen atom or a lower alkyl, lower alkoxy, hydroxy, trifluoromethyl or nitro group.

2. Therapeutic composition as claimed in claim 1, formulated in a form suitable for oral, parenteral or rectal administration, the active ingredient being combined with a therapeutically acceptable carrier.

3. Therapeutic composition as claimed in any one of the claims 1 and 2, in unit dosage form, each unit dose containing 0.40—0.750 g active ingredient.

4. Thieno- or furopyridone derivatives having the following general formulae:

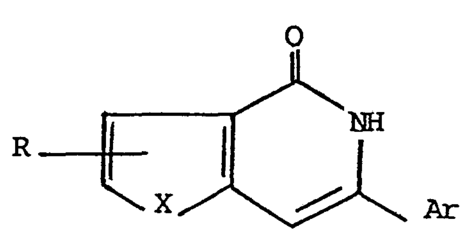

(I)

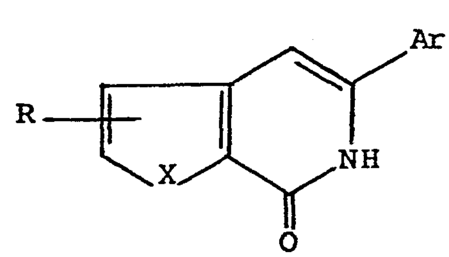

(II)

in which X is an oxygen or sulfur atom, R represents a hydrogen or halogen atom or a $C_{1-4}$ alkyl group, and Ar represents a phenyl, naphthyl or pyridyl group optionally substituted with at least a halogen atom or a lower alkyl, lower alkoxy, hydroxy, trifluoromethyl or nitro group, provided R = H, X = S and Ar = phenyl are not simultaneously present.

5. Process for the preparation of thienopyridone or furopyridone derivatives having the following general formulae:

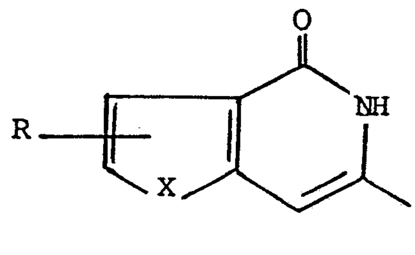

(I)

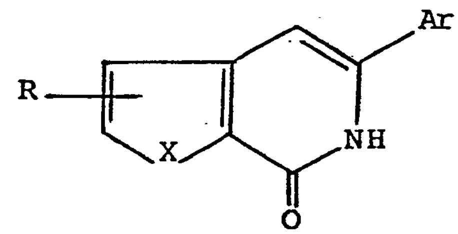

(II)

in which X is an oxygen or sulfur atom, R represents a hydrogen or halogen atom or a $C_{1-4}$ alkyl group, and Ar represents a phenyl, naphthyl or pyridyl group optionally substituted with at least a halogen atom or a lower alkyl, lower alkoxy, hydroxy, trifluoromethyl or nitro group, comprising heating within an inert solvent and at a temperature between 150°C and the boiling point of the solvent selected, a compound of the following formulae (III) or (IV):

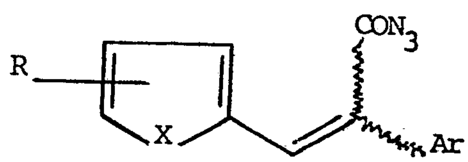

or

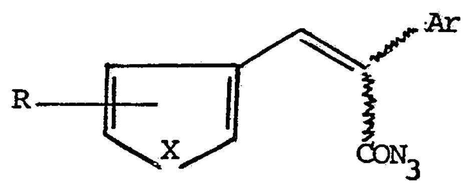

(III)

(IV)

in which X, R and Ar have the above-defined meanings.

6. Process as claimed in claim 5, wherein the inert solvent is selected from diphenylmethane and diphenyl ether.